# EUROPÄISCHE PATENTSCHRIFT

(11) **EP 4 169 416 B1**
(45) Veröffentlichungstag und Bekanntmachung des Hinweises auf die Patenterteilung: **04.12.2024**
(21) Anmeldenummer: 22202047.1
(22) Anmeldetag: 18.10.2022
(51) Int. Cl.: A45F 3/14, A61F 5/02

(54) **VORRICHTUNG ZUR UNTERSTÜTZUNG BEIM TRAGEN VON LASTEN**
DEVICE FOR ASSISTING IN CARRYING LOADS
DISPOSITIF D'ASSISTANCE AU TRANSPORT DE CHARGES

(30) Priorität: 21.10.2021 DE 102021127397
(43) Veröffentlichungstag der Anmeldung: 26.04.2023
(73) Patentinhaber: HUNIC GmbH, 72270 Baiersbronn (DE)
(72) Erfinder: Mast, Jonas, 72270 Baiersbronn (DE)
(74) Vertreter: Westphal, Mussgnug & Partner Patentanwälte mbB

(56) Entgegenhaltungen:
- EP-A1- 2 810 579
- WO-A2-2012/152863
- US-A1- 2020 103 073

## Beschreibung

Die Erfindung betrifft eine Vorrichtung zur Unterstützung beim Tragen von Lasten mit einem Hüftgurt gemäß dem Oberbegriff des Anspruchs 1.

Es ist bekannt, dass das manuelle Tragen von Lasten den Körper einer Person, insbesondere den Rücken einer Person, belasten kann. Daher sind Vorrichtungen zur Unterstützung beim Tragen von Lasten bekannt. Die DE 20 2007 014 U1 offenbart einen Tragegurt, welcher an einer Transportbox oder einem Getränkekasten befestigt werden kann und über die Schulter legbar ist, um das Gewicht der Transportbox oder des Getränkekastens auf die Schulter zu verlagern. Die DE 20 2018 107 422 U1 offenbart eine Tragehilfe mit einem Winkelelement, welches mittels eines Gurts über der Schulter getragen werden kann und das Gewicht einer zu tragenden Last auf die Schulter verlagert. Durch die Verlagerung der Gewichtskraft auf die Schulter wird jedoch die Wirbelsäule zusätzlich belastet.

Die DE 299 13 362 U1 offenbart beispielsweise eine Tragehilfe mit einem um die Hüfte tragbaren Gurt, welcher mittels Schlaufen die Last auf die Schultern einer tragenden Person führt, wobei eine bewegliche Frontklappe um unteren Bauchbereich der Person nach vorne ausklappbar ist. Auf die Frontklappe ist eine Last auflegbar, wodurch das tragen schwerer Lasten unterstützt werden kann. Auch bei dieser Tragehilfe wird das Gewicht auf die Schultern verlagert.

Die WO 2015/066793 A1 offenbart einen weiteren Hüftgurt zur Unterstützung beim Tragen von Lasten. Außerdem wird die EP 2 810 579 A1 als Stand der Technik genannt.

Die Aufgabe der Erfindung besteht darin, eine Vorrichtung zur Unterstützung beim Tragen von Lasten bereitzustellen, welche die Wirbelsäule beim Tragen von Lasten besser entlastet und zudem das Tragen von Lasten auch mit nur einer Hand ermöglicht.

Die Aufgabe der Erfindung wird gelöst durch eine Vorrichtung zur Unterstützung beim Tragen von Lasten mit den Merkmalen des Anspruchs 1.

Vorteilhafte Ausgestaltungen und Weiterbildungen der Erfindung sind in den abhängigen Ansprüchen angegeben.

Eine erfindungsgemäße Vorrichtung zur Unterstützung beim Tragen von Lasten mit einem Hüftgurt mit einer Innenseite zur Anlage an einen Körper einer Person und einer Außenseite, mit einem ersten Ende und einem zweiten Ende, welche mittels eines Verschlusselements miteinander verbindbar sind, zeichnet sich dadurch aus, dass der Hüftgurt ein Rückenelement zur Anlage an einen Abschnitt eines Rückens der Person aufweist, und dass an dem Rückenelement ein erster Hüftgurtabschnitt angeordnet ist, wobei an der Außenseite des ersten Hüftgurtabschnitts wenigstens ein, vorzugsweise mehrere, nach außen vorstehendes erstes Ablageelement in einem derartigen Abstand zum Rückenelement angeordnet ist, dass es bei Anlage an den Körper der Person seitlich an dem Körper der Person angeordnet ist. Ein vorstehendes Ablageelement ermöglicht die Anlage einer Last ohne einen zusätzlichen Handgriff. Die Anordnung eines Ablageelements seitlich am Körper der Person ermöglicht die Ablage einer Last auf der Hüfte, wobei die Gewichtskraft auf die Hüfte abgeleitet wird. Eine zusätzliche Belastung der Wirbelsäule oder der Schultern kann dadurch vermieden werden. Durch die Ablagemöglichkeit an der Hüfte wird der Schwerpunkt in der Regel weniger stark verschoben, sodass die Bildung eines Hohlkreuzes verringert werden kann. Durch die Anordnung des Ablageelements seitlich am Körper der Person wird zudem ein Balancestabilisiertes Tragen mit nur einer Hand möglich.

Eine besonders bevorzugte Weiterbildung der Erfindung sieht vor, dass an dem Rückenelement ein zweiter Hüftgurtabschnitt angeordnet ist, wobei an der Außenseite des zweiten Hüftgurtabschnitts wenigstens ein, vorzugsweise mehrere, nach außen vorstehendes zweites Ablageelement in einem derartigen Abstand zum Rückenelement angeordnet ist, dass es bei Anlage an den Körper der Person seitlich an dem Körper der Person und insbesondere auf der anderen Seite als das erste Ablageelement angeordnet ist. Dies ermöglicht die Unterstützung des Tragens einer Last sowohl auf der einen Seite als auch auf der anderen Seite einer Person. Eine einseitige Belastung der Person beim Tragen von Lasten kann dadurch vermieden werden.

Wenn im nachfolgenden von einem Hüftgurtabschnitt die Rede ist, sollen der erste Hüftgurtabschnitt und/oder der zweite Hüftgurtabschnitt damit umfasst sein. Ebenso sollen das erste Ablageelement und/oder das zweite Ablageelement umfasst sein, wenn im nachfolgenden von einem Ablageelement die Rede ist.

Vorzugsweist ist das Ablageelement aus einem Hartschaumstoff, insbesondere aus einem Hartschaumstoff mit einer Festigkeit von mehr als 55 Shore, gefertigt. Ein Hartschaumstoff ermöglicht eine einfache Fertigung. Zudem kann sich ein derartiger Hartschaumstoff bis zu einem gewissen Grad an die Anatomie des Körpers der Person anpassen. Andererseits kann der Hartschaumstoff das Tragen der Last ausreichend unterstützen.

Eine vorteilhafte Ausgestaltung der Erfindung sieht vor, dass das Ablageelement lösbar an dem Hüftgurtabschnitt angeordnet ist. Dadurch kann auf einfache Art und Weise ein Auswechseln des Ablageelements ermöglicht werden, beispielsweise um für unterschiedliche zu tragende Lasten jeweils ein geeignetes Ablageelement verwenden zu können.

Vorzugsweise ist das Ablageelement mittels eines Hakens in eine entsprechende Ausnehmung an dem Hüftgurtabschnitt einhängbar oder das Ablageelement mittels einer Schwalbenschwanzführung an dem Hüftgurtabschnitt befestigbar. Diese beiden Befestigungsmöglichkeiten ermöglichen eine zuverlässige Befestigung und ein einfaches Auswechseln.

Besonders bevorzugt ist das Verschlusselement als Reißverschluss ausgebildet. Ein derartiges Verschlusselement ist leicht zu öffnen und zu schließen und trägt möglichst wenig auf.

Vorteilhafterweise ist der Hüftgurt in der Weite verstellbar, um eine Anpassung an unterschiedliche anatomische Ausgestaltungen von Personen zu ermöglichen.

Gemäß einer bevorzugten Ausführungsform der Erfindung sind wenigstens der erste Hüftgurtabschnitt, vorzugsweise der erste Hüftgurtabschnitt und der zweite Hüftgurtabschnitt, lösbar an dem Rückenelement angeordnet. Einerseits kann eine derartige Ausgestaltung eine Trennung zu Reinigungszwecken ermöglichen. Andererseits kann eine derartige Ausgestaltung eine Verstellbarkeit der Weite des Hüftgurts ermöglichen.

Vorzugsweise ist wenigstens der erste Hüftgurtabschnitte, vorzugsweise der erste Hüftgurtabschnitt und der zweite Hüftgurtabschnitt, mit einem freien Ende durch eine an dem Rückenelement angeordnete Schlinge geführt, wobei das freie Ende in unterschiedlichen Positionen an einem Befestigungsabschnitt des Hüftgurtabschnitts fixierbar ist, vorzugsweise mittels einer Klett-Flausch-Verbindung. Eine derartige Ausgestaltung ermöglicht die Variation der Weite des Hüftgurts auf einfache Art und Weise.

Vorteilhafterweise weist das Rückenelement eine größere Höhe auf als der Hüftgurt, wodurch eine gute Stabilisierung des Rückens ermöglicht werden kann.

Eine besonders bevorzugte Weiterbildung der Erfindung sieht vor, dass zwischen einem oberen Abschnitt des Rückenelements und dem ersten Hüftgurtabschnitt und/oder dem zweiten Hüftgurtabschnitt ein Stützgurt angeordnet ist, welcher insbesondere in seiner Länge verstellbar ist, vorzugsweise indem er mit einem freien Ende durch eine an dem Rückenelement angeordnete Schlinge geführt ist, wobei das freie Ende in unterschiedlichen Positionen an einem Befestigungsabschnitt des Stützgurts fixierbar ist, vorzugsweise mittels einer Klett-Flausch-Verbindung. Ein derartiger Stützgurt kann die auf den Hüftgurt insbesondere im Bereich der Hüfte wirkenden Kräfte auf das Rückenelement verteilen und der Bildung eines Hohlkreuzes entgegenwirken.

Vorzugsweise ist die erfindungsgemäße Vorrichtung erweiterbar, um weitere Unterstützungsfunktionen zu ermöglichen, beispielsweise im Rahmen eines Exoskeletts. Vorteilhafterweise sind ein oberes Rückenelement und/oder zwei Schultergurte und/oder zwei Beinbänder insbesondere lösbar an der Vorrichtung angeordnet, um derartige erweiterte oder zusätzliche Funktionen ermöglichen zu können.

Die Erfindung wird anhand der nachfolgenden Figuren ausführlich erläutert. Es zeigen
Figur 1a eine perspektivische Ansicht eines ersten Ausführungsbeispiels einer erfindungsgemäßen Vorrichtung zur Unterstützung beim Tragen von Lasten,
Figur 1b die Vorrichtung gemäß Figur 1a im an eine Person angelegten Zustand,
Figur 2a eine weitere perspektivische Ansicht der Vorrichtung gemäß Figur 1a,
Figur 2b die Vorrichtung gemäß Figur 2a im an eine Person angelegten Zustand,
Figur 3a eine Ansicht von vorne auf die Vorrichtung gemäß Figur 1a,
Figur 3b die Vorrichtung gemäß Figur 3a im an eine Person angelegten Zustand,
Figur 4a eine Ansicht von hinten auf die Vorrichtung gemäß Figur 1a,
Figur 4b die Vorrichtung gemäß Figur 4a im an eine Person angelegten Zustand,
Figur 5a eine Seitenansicht der Vorrichtung gemäß Figur 1a,
Figur 5b die Vorrichtung gemäß Figur 5a im an eine Person angelegten Zustand,
Figur 6 die Person gemäß Figur 5b mit einer Last,
Figur 7 eine Explosionsdarstellung der Vorrichtung gemäß Figur 1a,
Figur 8 eine Ansicht von vorne auf ein zweites Ausführungsbeispiel einer erfindungsgemäßen Vorrichtung zur Unterstützung beim Tragen von Lasten mit Erweiterungen zu einem Exoskelett,
Figur 9 eine Ansicht von hinten auf das Exoskelett gemäß Figur 8,
Figur 10 eine perspektivische Ansicht des Exoskeletts gemäß Figur 8,
Figur 11 eine Explosionsdarstellung des Exoskeletts gemäß Figur 8 und
Figur 12 das Exoskelett gemäß Figur 8 im an eine Person angelegten Zustand.

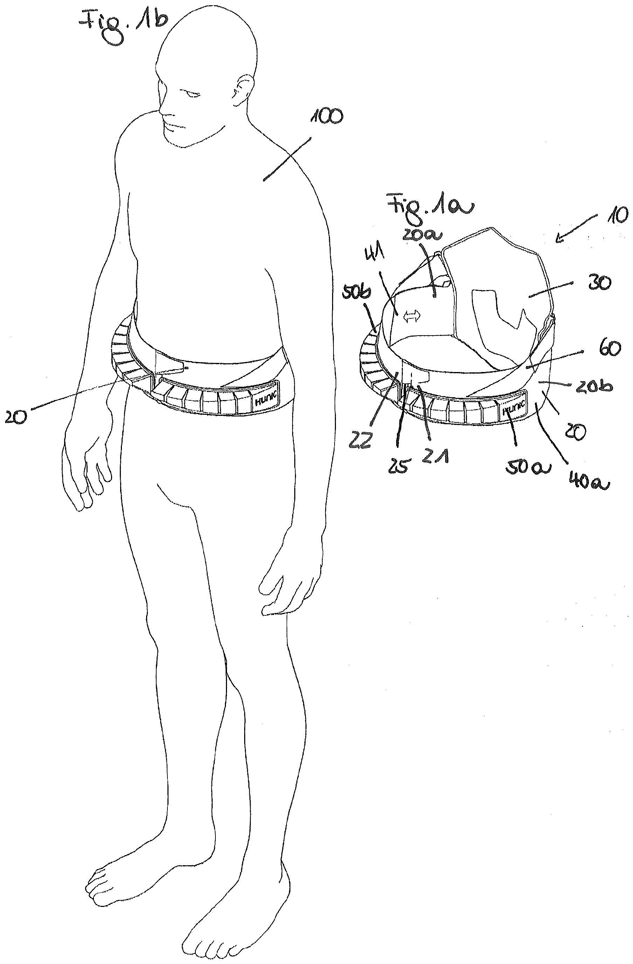

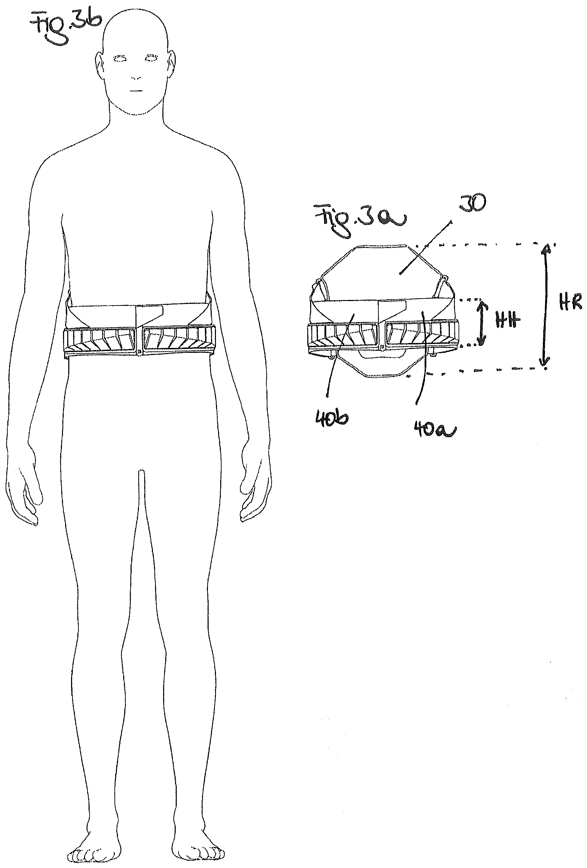

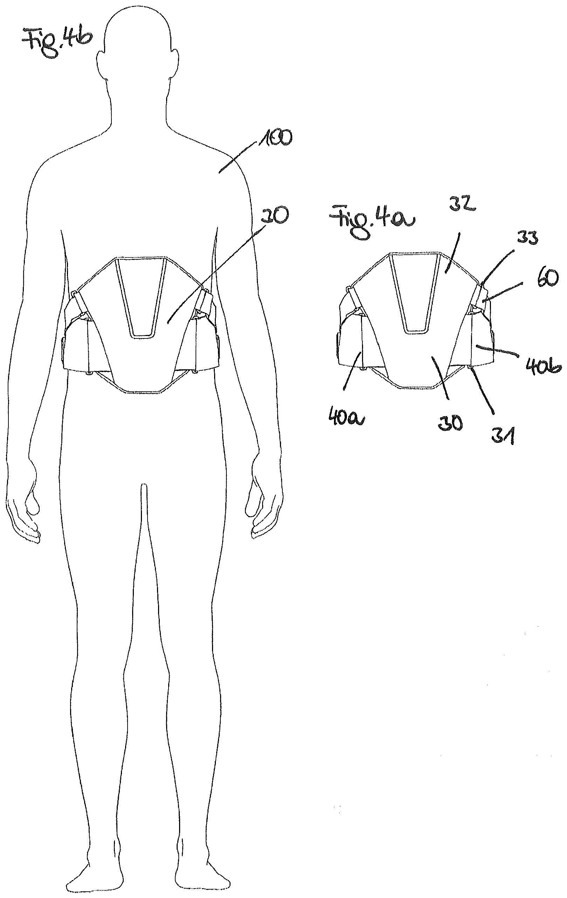

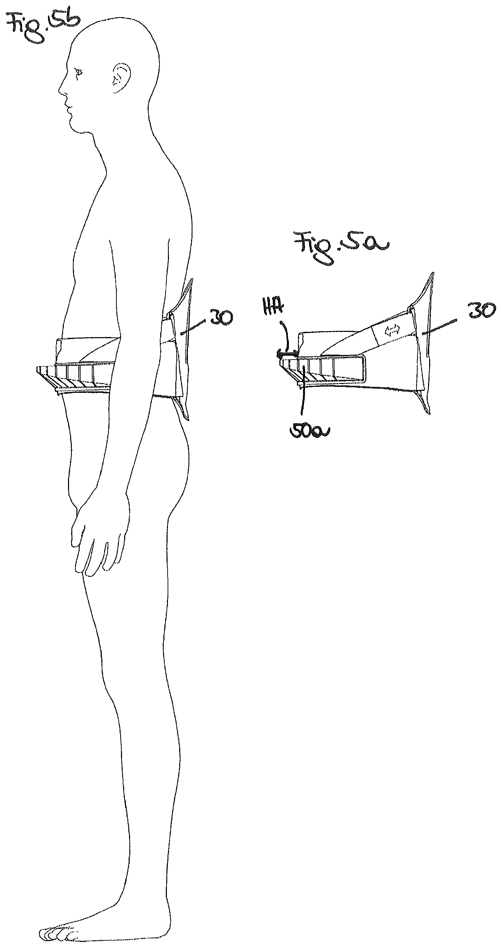

Die Figuren 1a bis 7 zeigen verschiedene Ansichten eines ersten Ausführungsbeispiels einer Vorrichtung 10 zur Unterstützung beim Tragen von Lasten, teils separat und teils im an eine Person 100 angelegten Zustand. Gleiche Bezugsziffern bezeichnen in allen Figuren gleiche oder funktionsgleiche Teile. Zur besseren Übersicht sind nicht sämtliche Bezugsziffern in sämtlichen Figuren angegeben.

Die Vorrichtung 10 weist einen Hüftgurt 20 mit einer Innenseite 20a zur Anlage an einen Körper einer Person 100 und einer Außenseite 20b auf. Der Hüftgurt 20 ist dabei insbesondere als im wesentlichen langgestrecktes Element ausgebildet, welches bestimmungsgemäß um die Taille der Person 100 gelegt wird und dabei insbesondere auf der Hüfte der Person 100 aufliegt (vgl. Figur 1b). Der Hüftgurt 20 weist ferner ein erstes Ende 21 und ein zweites Ende 22 auf, welche mittels eines Verschlusselements 25 miteinander verbindbar sind. Das Verschlusselement 25 kann beispielsweise als Klettverschluss ausgebildet sein. Bevorzugt ist das Verschlusselement 25 als Reißverschluss ausgebildet, um ein möglichst passgenaues Sitzen des Hüftgurts 20 ohne auftragende Elemente ermöglichen zu können.

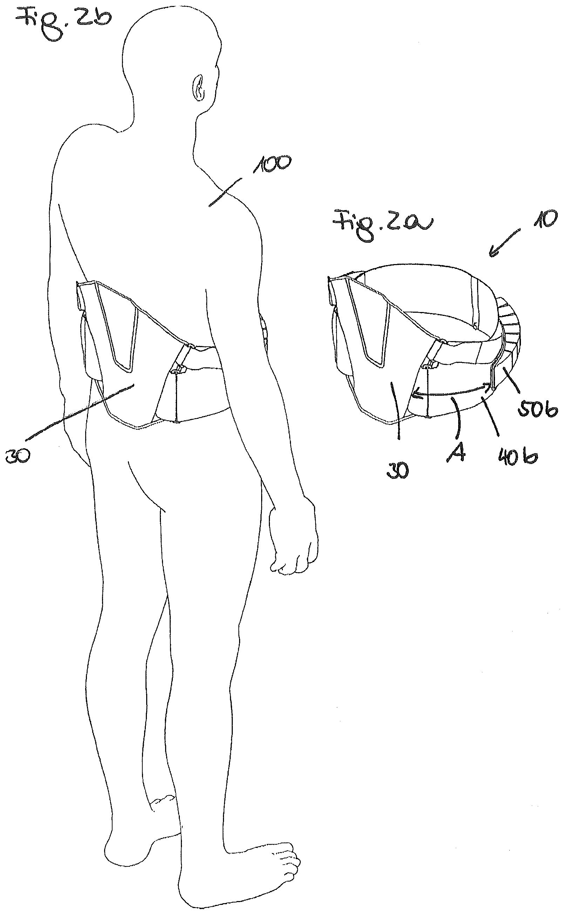

Der Hüftgurt 20 weist ein Rückenelement 30 zur Anlage an einen Abschnitt eines Rückens der Person 100 auf (vgl. insbesondere Figuren 2a und 2b). Das Rückenelement 30 bildet insbesondere einen versteiften Abschnitt zur Unterstützung der Wirbelsäule und um der Bildung eines Hohlkreuzes bei Belastung entgegen wirken zu können. Das Rückenelement 30 kann beispielsweise als Hartschale ausgebildet sein. Dadurch kann das Rückenelement 30 mit einer möglichst geringen Wandstärke von weniger als 2 cm, vorzugsweise weniger als 1 cm, ausgebildet werden, um möglichst wenig aufzutragen.

Das Rückenelement 30 kann zumindest abschnittsweise, vorzugsweise vollständig, aus einem formelastischen Material gefertigt. Das formelastische Material kann beispielsweise Metall oder Kunststoff sein, wobei Kunststoff aufgrund des geringeren Gewichts in der Regel bevorzugt wird. Das formelastische Material weist vorzugsweise ein E-Modul von mehr als 600 N/mm², besonders bevorzugt ein E-Modul von mehr als 1000 N/mm², beispielsweise ein E-Modul von 5000 N/mm² auf. Ist das Rückenelement 30 abschnittsweise aus einem formelastischen Material gefertigt, kann es beispielsweise aus einem Textilgewebe mit einem eingelegten Element aus einem formelastischen Material gefertigt sein.

An dem Rückenelement 30 ist ein erster Hüftgurtabschnitt 40a angeordnet. Der erste Hüftgurtabschnitt 40a kann aus einem elastischen Textilgewebe gefertigt sein. Der erste Hüftgurtabschnitt 40a umfasst das erste Ende 21 des Hüftgurts 20 und weist zudem ein freies Ende 41 auf, mit welchem der erste Hüftgurtabschnitt 40a an dem Rückenelement 30 befestigt ist. Es ist möglich, dass das zweite Ende 22 an dem Rückenelement 30 ausgebildet ist und das erste Ende 21 zum Schließen des Hüftgurts 20 an dem am Rückenelement 30 angeordneten zweiten Ende 22 befestigt wird. Vorzugsweise ist jedoch an dem Rückenelement 30 ein zweiter Hüftgurtabschnitt 40b angeordnet. Der zweite Hüftgurtabschnitt 40b kann aus einem elastischen Textilgewebe gefertigt sein. Der zweite Hüftgurtabschnitt 40b umfasst das zweite Ende 22 des Hüftgurts 20 und weist zudem ein freies Ende 41 auf, mit welchem der zweite Hüftgurtabschnitt 40b an dem Rückenelement befestigt ist. Eine derartige Ausgestaltung ermöglicht insbesondere die Anordnung des Verschlusselements 25 in etwa im Bereich vor dem Bauchnabel der Person 100. Weiterhin ermöglicht eine derartige Ausgestaltung insbesondere einen symmetrischen Aufbau des Hüftgurts 20.

Der Hüftgurt 20 kann in der Weite verstellbar sein. Dies kann durch eine entsprechende Ausgestaltung des Verschlusselements 25 beispielsweise nach Art eines Gürtels oder mittels eines Klettverschlusses realisiert sein.

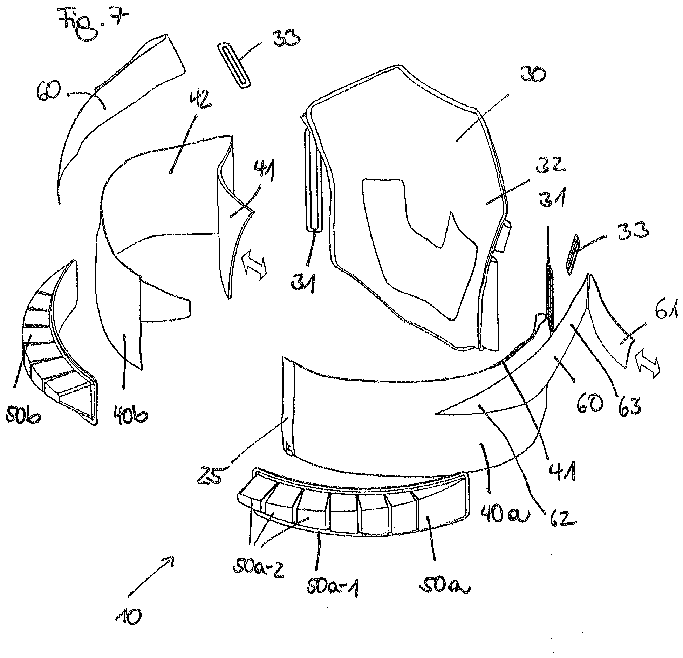

Alternativ oder zusätzlich kann wenigstens der erste Hüftgurtabschnitt 40a, vorzugsweise der erste Hüftgurtabschnitt 40a und der zweite Hüftgurtabschnitt 40b, lösbar an dem Rückenelement 30 angeordnet sein. Dazu kann das freie Ende 41 des Hüftgurtabschnitts 40a, 40b durch eine an dem Rückenelement 30 angeordnete Schlinge 31 geführt sein. Die Schlinge 31 kann beispielsweise als Metallring, welcher insbesondere eine langgestreckte Form aufweist, ausgebildet sein (vgl. insbesondere Figur 7). Das freie Ende 41 kann in unterschiedlichen Positionen an einem Befestigungsabschnitt 42 des Hüftgurtabschnitts 40a, 40b fixierbar sein. Dazu kann vorzugsweise eine Klett-Flausch-Verbindung vorgesehen sein, wobei an dem freien Ende 41 ein Klettabschnitt und an dem Befestigungsabschnitt 42 ein Flauschabschnitt oder umgekehrt vorgesehen sein können. Auf diese Weise lässt sich die Weite einfach durch die Person 100 variieren. Sind beide Hüftgurtabschnitte 40a, 40b derart lösbar an dem Rückenelement 30 befestigt, kann die Weite insbesondere derart variiert werden, dass das Verschlusselement 25 für alle Weiten im Bereich des Bauchnabels der Person 100 angeordnet ist und eine symmetrische Anordnung des Hüftgurts 20 an der Person 100 möglich ist.

An der Außenseite 20b des ersten Hüftgurtabschnitts 40a ist wenigstens ein nach außen vorstehendes erstes Ablageelement 50a in einem derartigen Abstand A zum Rückenelement 30 angeordnet ist, dass es bei Anlage an den Körper der Person 100 seitlich an dem Körper der Person 100 angeordnet ist, insbesondere etwa im Bereich oberhalb des Beckenknochens. Das Ablageelement 50a kann sich entlang des Hüftgurtabschnitts 40a bis in die Nähe des Verschlusselements 25 erstrecken. Das Ablageelement 50a kann in Richtung senkrecht zur Außenseite 20b des Hüftgurts 20 eine Höhe HA von einigen Zentimetern, beispielsweise im Bereich von 3 cm bis 15 cm, insbesondere im Bereich von 6 cm bis 10 cm, aufweisen. Das Ablageelement 50a kann dabei an einem Basiselement 50a-1 mehrere Vorsprünge 50a-2 nebeneinander angeordnet sein, um eine bessere Anpassung des Ablageelements 50a an unterschiedliche Weiten des Hüftgurts 20 ermöglichen zu können. Es können auch mehrere einzelne Ablageelemente 50a in diesem Bereich nebeneinander angeordnet sein. Vorteilhaft ist, dass sich das Ablageelement 50a im an die Person 100 angelegten Zustand im Bereich oberhalb des Beckenknochens befindet, um eine Anlage einer mit einem Arm zu tragenden Last im Bereich der Hüfte ermöglichen zu können.

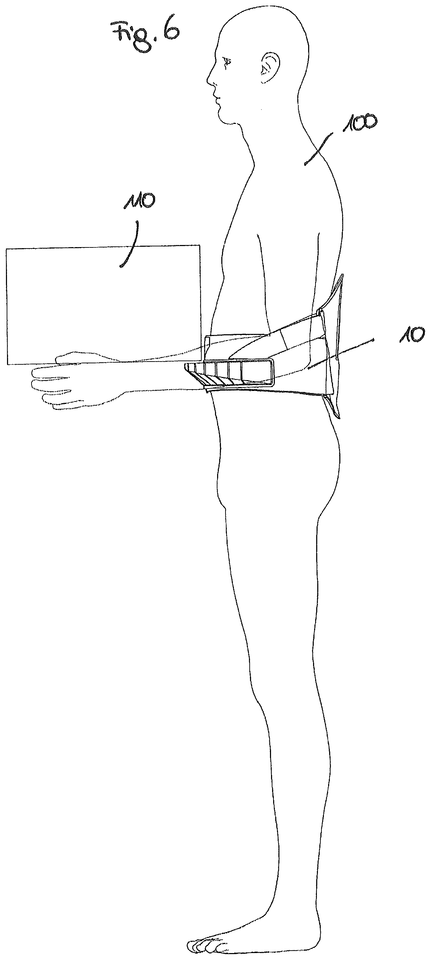

Wie in Figur 6 erkennbar, ermöglicht die Ausgestaltung derart, dass das Ablageelement 50a, 50b sich bis in den Bereich vor dem Bauch der Peron 100 erstreckt, einerseits das beidhändige Tragen einer Last 110 mit Abstützung an dem Ablageelement 50a, 50b vor dem Bauch, andererseits aber auch das einhändige Tragen der Last 110 mit Abstützung entweder an dem Ablageelement 50a an der linken Seite der Person 100 oder mit Abstützung an dem Ablageelement 50b an der rechten Seite der Person 100.

Das Ablageelement 50a kann aus einem Hartschaumstoff, insbesondere aus einem Hartschaumstoff mit einer Festigkeit von mehr als 55 Shore, gefertigt sein.

Das Ablageelement 50a kann lösbar an dem Hüftgurtabschnitt 40a angeordnet sein. Dazu kann beispielsweise das Ablageelement 50a mittels eines Hakens in eine entsprechende Ausnehmung an dem Hüftgurtabschnitt 40a einhängbar sein oder das Ablageelement 50a mittels einer Schwalbenschwanzführung an dem Hüftgurtabschnitt 40a befestigbar sein.

Sofern ein zweiter Hüftgurtabschnitt 40b vorhanden ist, ist an der Außenseite 20b des zweiten Hüftgurtabschnitts 40b wenigstens ein, vorzugsweise mehrere, nach außen vorstehendes zweites Ablageelement 50b in einem derartigen Abstand A zum Rückenelement 30 angeordnet ist, dass es bei Anlage an den Körper der Person 100 seitlich an dem Körper der Person 100 und insbesondere auf der anderen Seite als das erste Ablageelement 50a angeordnet ist. Ansonsten gelten für das zweite Ablageelement 50b die zuvor zu dem ersten Ablageelement 50a gemachten Ausführungen.

Das Rückenelement 30 kann eine größere Höhe HR aufweisen als der Hüftgurt 20. Beispielsweise kann der Hüftgurt 20 eine Höhe HH im Bereich von etwa 10 cm bis 15 cm, beispielsweise eine Höhe HH von etwa 12 cm, aufweisen. Das Rückenelement 30 kann eine Höhe HR von 20 cm bis 35 cm aufweisen.

Zwischen einem oberen Abschnitt 32 des Rückenelements 30 und dem ersten Hüftgurtabschnitt 40a ist ein Stützgurt 60 angeordnet, welcher insbesondere in seiner Länge verstellbar ist. Der Stützgurt weist ein freies Ende 61 auf, welches insbesondere durch eine an dem Rückenelement 30 angeordnete Schlinge 33 geführt sein kann, wobei das freie Ende 61 in unterschiedlichen Positionen an einem Befestigungsabschnitt 63 des Stützgurts fixierbar ist, vorzugsweise mittels einer Klett-Flausch-Verbindung. Die Schlinge 33 kann beispielsweise als Metallring, welcher insbesondere eine langgestreckte Form aufweist, ausgebildet sein (vgl. insbesondere Figur 7). Mit einem fixierten Ende 62 kann der Stützgurt 60 an dem ersten Hüftgurtabschnitt 40a fixiert sein.

Ebenso kann zwischen dem oberen Abschnitt 32 des Rückenelements 30 und dem zweiten Hüftgurtabschnitt 40b ein ebensolcher Stützgurt 60 angeordnet sein.

Die Vorrichtung 10 kann durch zusätzliche Komponenten zu einem Exoskelett erweitert werden.

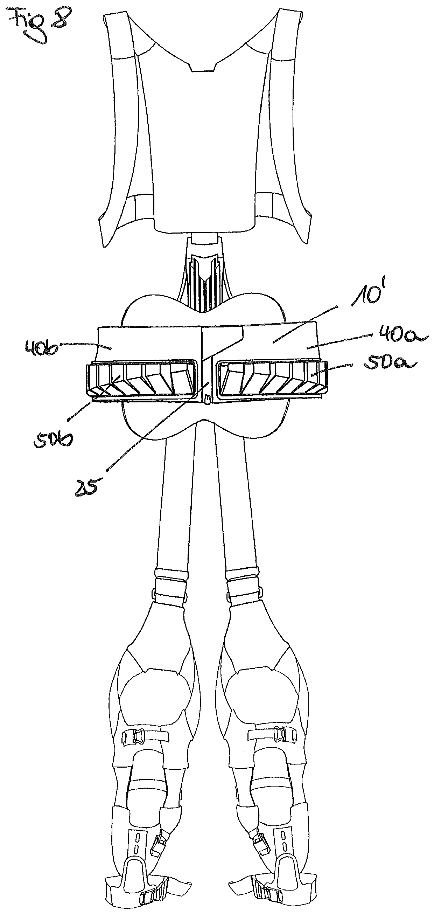

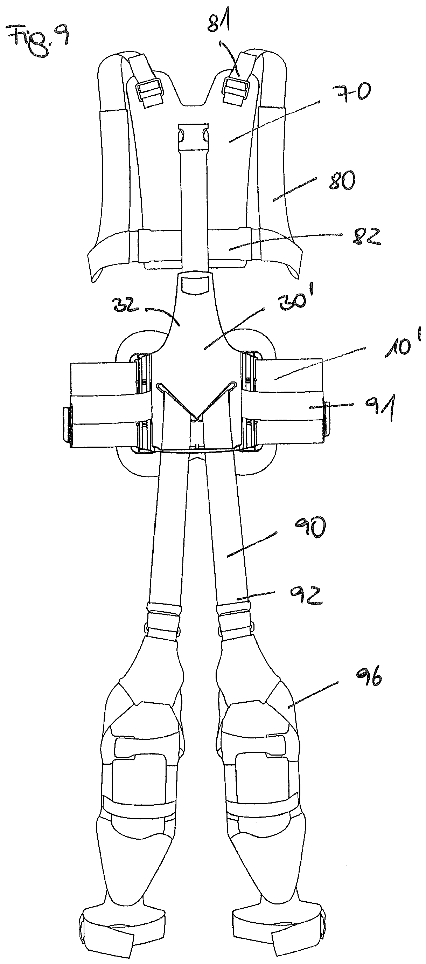

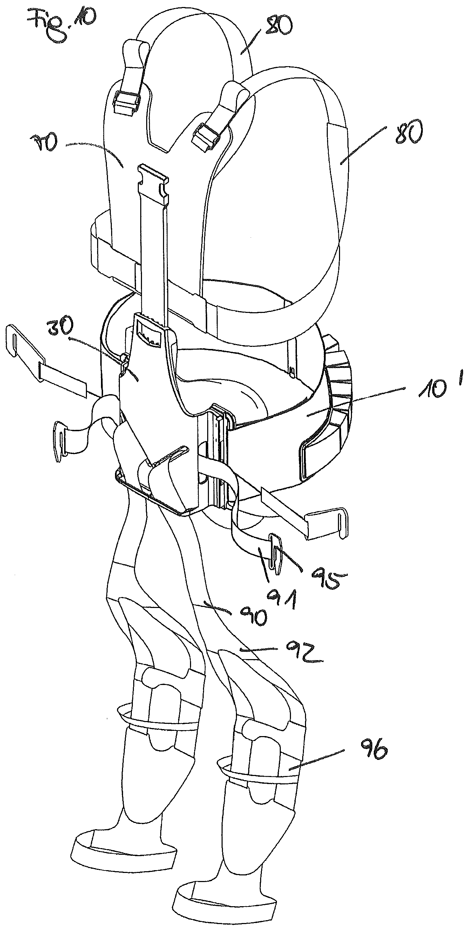

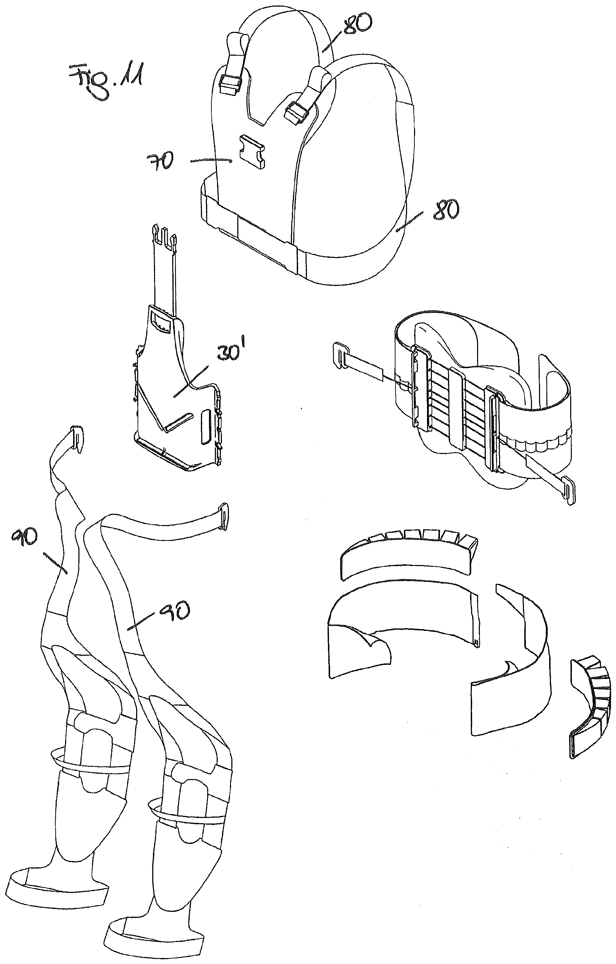

Die Figuren 8 bis 12 zeigen verschiedene Ansichten eines zweiten Ausführungsbeispiels einer Vorrichtung 10` zur Unterstützung beim Tragen von Lasten, welches mit zusätzlichen Komponenten zu einem Exoskelett erweitert wurde. Die Vorrichtung 10` unterscheidet sich dabei von der zuvor anhand der Figuren 1 bis 7 beschriebenen Vorrichtung 10 im Wesentlichen durch die Ausgestaltung des Rückenelements 30 und dadurch, dass keine Stützgurte 60 vorhanden sind.

Die Vorrichtung 10` weist ein Rückenelement 30' auf, welches in seinem oberen Abschnitt eine Anbindung an ein oberes Rückenelement 70 ermöglichen kann. Das obere Rückenelement 70 kann dabei eine derartige Länge aufweisen, dass es gemeinsam mit dem Rückenelement 30' ein Element bildet, das sich im an die Person 100 angelegten Zustand vom Bereich der Hüfte bis in den Bereich des oberen Rückens oder Nackens erstreckt. Insbesondere kann das obere Rückenelement 70 auch an seinem dem Hüftgurt 20 abgewandten Ende auf den Schultern der Person 100 aufliegen. Das Rückenelement 30' und das obere Rückenelement 70 können in der Länge verstellbar gegeneinander fixierbar sein.

Das obere Rückenelement 70 kann zwei Schulterbänder 80 aufweisen, welche jeweils ein erstens Ende 81 und ein zweites Ende 82 aufweisen. Als Schulterbänder 80 sollen im Folgenden die Elemente verstanden werden, die das obere Rückenelement 70 im an die Person 100 angelegten Zustand des oberen Rückenelements 70 am Rücken der Person anliegend fixieren. Die Schulterbänder 80 können längenverstellbar an dem Rückenelement 30` angeordnet sein. Die Schulterbänder 80 können aus einem Gurtmaterial, beispielsweise aus einem Gewebeband, gefertigt sein.

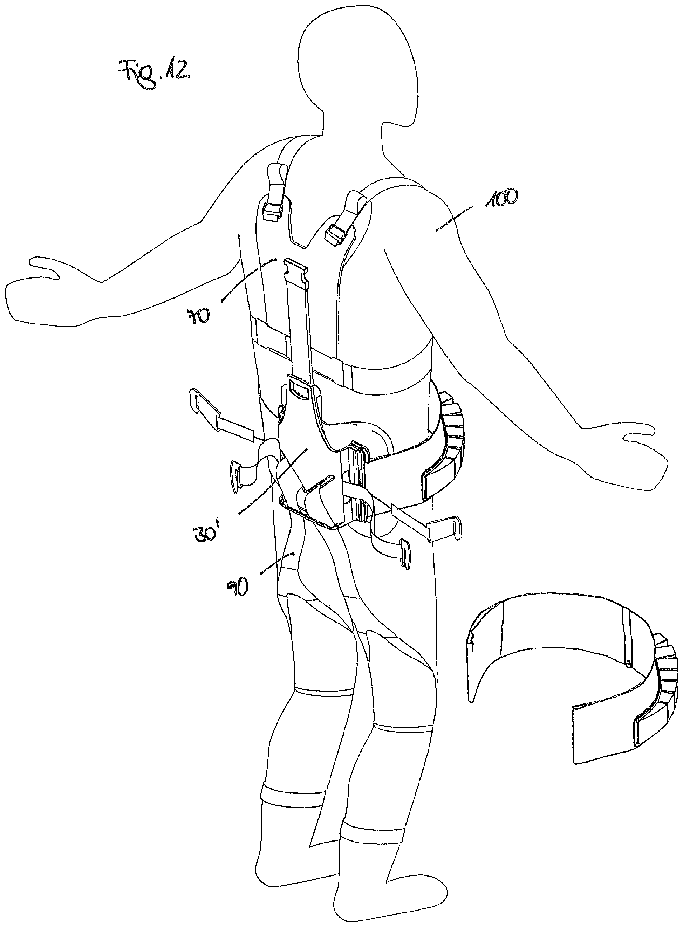

An dem Hüftgurt 20, insbesondere an dem Rückenelement 30`, können zusätzlich oder alternativ zu dem oberen Rückenelement 70 zwei Beinbänder 90 angeordnet sein, insbesondere derart, dass sie im an die Person 100 angelegten Zustand der Vorrichtung ausgehend von der Hüfte über das Gesäß der Person 100 an den Beinen anliegend verlaufen (siehe Figur 12). Die Beinbänder 90 weisen ein erstes Ende 91 und ein zweites Ende 92 auf. Die Beinbänder 90 sind vorzugsweise lösbar mit dem ersten Ende 91 an dem Hüftgurt 20 angeordnet, insbesondere an der Außenseite 20b des Hüftgurts 20, beispielsweise mittels eines Hakens 95, einer Schnappschnalle oder alternativ nach Art eines Verschluss eines Gürtels oder mittels eines Klettverschlusses oder sonstigen Verschlusses. Die Beinbänder 90 sind insbesondere längenverstellbar ausgebildet.

An dem zweiten Ende 92 des Beinbands 90 kann ein Knieelement 96 angeordnet sein, um das Beinband 90 fixieren zu können.

Die Beinbänder 90 und/oder das Knieelement 96 können zumindest abschnittsweise aus einem Elastomer gefertigt sein. Das Elastomer kann insbesondere ein E-Modul von weniger als 600 N/mm², bevorzugt ein E-Modul von weniger als 150 N/mm², besonders bevorzugt ein E-Modul von weniger als 100 N/mm², vorzugsweise ein E-Modul von etwa 30 N/mm² aufweisen.

Die Beinbänder 90 können das Aufrichten nach einem Bücken oder in die Hocke gehen der Person 100 unterstützen.

### Bezugszeichenliste

- 10: Vorrichtung
- 10': Vorrichtung
- 20: Hüftgurt
- 20a: Innenseite
- 20b: Außenseite
- 21: erstes Ende
- 22: zweites Ende
- 25: Verschlusselement
- 30: Rückenelement
- 30': Rückenelement
- 31: Schlinge
- 32: oberer Abschnitt
- 33: Schlinge
- 40a: erster Hüftgurtabschnitt
- 40b: zweiter Hüftgurtabschnitt
- 50a: erstes Ablageelement
- 50a-1: Basiselement
- 50a-2: Vorsprung
- 50b: zweites Ablageelement
- 41: freies Ende
- 42: Befestigungsabschnitt
- 60: Stützgurt
- 61: freies Ende
- 62: fixiertes Ende
- 70: oberes Rückenelement
- 80: Schultergurt
- 81: erstes Ende
- 82: zweites Ende
- 90: Beingurt
- 91: erstes Ende
- 92: zweites Ende
- 95: Haken
- 96: Knieelement
- 100: Person
- 110: Last
- A: Abstand
- HH: Höhe
- HR: Höhe
- HA: Höhe

## Patentansprüche

1. Vorrichtung (10, 10') zur Unterstützung beim Tragen von Lasten mit einem Hüftgurt (20) mit einer Innenseite (20a) zur Anlage an einen Körper einer Person (100) und einer Außenseite (20b), mit einem ersten Ende (21) und einem zweiten Ende (22), welche mittels eines Verschlusselements (25) miteinander verbindbar sind, wobei der Hüftgurt (20) ein Rückenelement (30, 30') zur Anlage an einen Abschnitt eines Rückens der Person (100) aufweist, und dass an dem Rückenelement (30, 30') ein erster Hüftgurtabschnitt (40a) angeordnet ist, **dadurch gekennzeichnet, dass** an der Außenseite (20a) des ersten Hüftgurtabschnitts (40a) wenigstens ein, vorzugsweise mehrere, nach außen vorstehendes erstes Ablageelement (50a) in einem derartigen Abstand (A) zum Rückenelement (30, 30') angeordnet ist, dass es bei Anlage an den Körper der Person (100) seitlich an dem Körper der Person (100) angeordnet ist.

2. Vorrichtung nach Anspruch 1,
**dadurch gekennzeichnet, dass** an dem Rückenelement (30, 30') ein zweiter Hüftgurtabschnitt (40b) angeordnet ist, wobei an der Außenseite (20b) des zweiten Hüftgurtabschnitts (40b) wenigstens ein, vorzugsweise mehrere, nach außen vorstehendes zweites Ablageelement (50b) in einem derartigen Abstand (A) zum Rückenelement (30, 30') angeordnet ist, dass es bei Anlage an den Körper der Person (100) seitlich an dem Körper der Person (100) und insbesondere auf der anderen Seite als das erste Ablageelement (50a) angeordnet ist.

3. Vorrichtung nach einem der vorhergehenden Ansprüche,
**dadurch gekennzeichnet, dass** das Ablageelement (50a, 50b) aus einem Hartschaumstoff, insbesondere aus einem Hartschaumstoff mit einer Festigkeit von mehr als 55 Shore, gefertigt ist.

4. Vorrichtung nach einem der vorhergehenden Ansprüche,
**dadurch gekennzeichnet, dass** das Ablageelement (50a, 50b) lösbar an dem Hüftgurtabschnitt (40a, 40b) angeordnet ist.

5. Vorrichtung nach einem der vorhergehenden Ansprüche,
**dadurch gekennzeichnet, dass** das Ablageelement (50a, 50b) mittels eines Hakens in eine entsprechende Ausnehmung an dem Hüftgurtabschnitt (40a, 40b) einhängbar ist oder dass das Ablageelement (50a, 50b) mittels einer Schwalbenschwanzführung an dem Hüftgurtabschnitt (40a , 40b) befestigbar ist.

6. Vorrichtung nach einem der vorhergehenden Ansprüche,
**dadurch gekennzeichnet, dass** das Verschlusselement (25) als Reißverschluss ausgebildet ist.

7. Vorrichtung nach einem der vorhergehenden Ansprüche,
**dadurch gekennzeichnet, dass** der Hüftgurt (20) in der Weite verstellbar ist.

8. Vorrichtung nach einem der vorhergehenden Ansprüche,
**dadurch gekennzeichnet, dass** wenigstens der erste Hüftgurtabschnitt (40a) , vorzugsweise der erste Hüftgurtabschnitt (40b) und der zweite Hüftgurtabschnitt (40b), lösbar an dem Rückenelement (30, 30') angeordnet sind.

9. Vorrichtung nach einem der vorhergehenden Ansprüche,
**dadurch gekennzeichnet, dass** wenigstens der erste Hüftgurtabschnitt (40a), vorzugsweise der erste Hüftgurtabschnitt (40a) und der zweite Hüftgurtabschnitt (40b), mit einem freien Ende (41) durch eine an dem Rückenelement (30, 30') angeordnete Schlinge (31) geführt ist, wobei das freie Ende (41) in unterschiedlichen Positionen an einem Befestigungsabschnitt (42) des Hüftgurtabschnitts (40a, 40b) fixierbar ist, vorzugsweise mittels einer Klett-Flausch-Verbindung.

10. Vorrichtung nach einem der vorhergehenden Ansprüche,
**dadurch gekennzeichnet, dass** das Rückenelement (30, 30') eine größere Höhe (HR) aufweist als der Hüftgurt (20).

11. Vorrichtung nach einem der vorhergehenden Ansprüche,
**dadurch gekennzeichnet, dass** zwischen einem oberen Abschnitt (32) des Rückenelements (30) und dem ersten Hüftgurtabschnitt (40a) und/oder dem zweiten Hüftgurtabschnitt (40b) ein Stützgurt (60) angeordnet ist, welcher insbesondere in seiner Länge verstellbar ist, vorzugsweise indem er mit einem freien Ende (61) durch eine an dem Rückenelement (30) angeordnete Schlinge (33) geführt ist, wobei das freie Ende (61) in unterschiedlichen Positionen an einem Befestigungsabschnitt (63) des Stützgurts (60) fixierbar ist, vorzugsweise mittels einer Klett-Flausch-Verbindung.

12. Vorrichtung nach einem der vorhergehenden Ansprüche,
**dadurch gekennzeichnet, dass** ein oberes Rückenelement (70) und/oder zwei Schultergurte (80) und/oder zwei Beinbänder (90) insbesondere lösbar an der Vorrichtung (10') angeordnet sind.

## Claims

1. Apparatus (10, 10') for rendering support while carrying loads, having a waist belt (20) with an inner side (20a) for application onto the body of a person (100) and with an outer side (20b), having a first end (21) and a second end (22) which are connected to each other by means of a locking element (25),
wherein the waist belt (20) comprises a back element (30, 30') for application onto a section of the back of the person (100) and a first waist belt section (40a) is arranged on the back element (30, 30'),
**characterized in that**, on the outer side (20b) of the first waist belt section (40a), at least one, preferably numerous, outwardly protruding first support elements (50a) is(are) arranged at such a distance (A) to the back element (30, 30') that, when worn on the body of the person (100), is arranged to one side on the body of the person (100).

2. Apparatus in accordance with claim 1,
**characterized in that** a second waist belt section (40b) is arranged on the back element (30, 30'), wherein, on the outer side (20b) of the second waist belt section (40b), at least one, preferably numerous outwardly protruding second support elements (50b) are arranged in such a distance (A) to the back element (30, 30') that, when worn on the body of the person (100), is arranged to one side on the body of the person (100) and, in particular, on the side opposite that of the first support element (50a).

3. Apparatus in accordance with either of the preceding claims,
**characterized in that** the support element (50a, 50b) is made of a rigid foam material, in particular, of a rigid foam material having a hardness of more than 55 Shore.

4. Apparatus in accordance with any of the preceding claims,
**characterized in that** the support element (50a, 50b) is detachably arranged on the waist belt section (40a, 40b).

5. Apparatus in accordance with any of the preceding claims,
**characterized in that** the support element (50a, 50b) can be suspended in a corresponding recess on the waist belt section (40a, 40b) by means of a hook or **in that** the support element (50a, 50b) can be attached to the waist belt section (40a, 40b) by means of a dovetail guide.

6. Apparatus in accordance with any of the preceding claims,
**characterized in that** the locking element (25) is implemented as a zip fastener.

7. Apparatus in accordance with any of the preceding claims,
**characterized in that** the waist belt (20) is adjustable in width.

8. Apparatus in accordance with any of the preceding claims,
**characterized in that** at least the first waist belt section (40a), preferably the first waist belt section (40a) and the second waist belt section (40b), is(are) detachably arranged on the back element (30, 30').

9. Apparatus in accordance with any of the preceding claims,
**characterized in that** a free end (41) of at least the first waist belt section (40a), preferably of the first waist belt section (40a) and of the second waist belt section (40b) is fed through a loop (31) arranged on the back element (30, 30'), wherein the free end (41) can be secured in various positions on an attachment section (42) of the waist belt section (40a, 40b), preferably by means of a hook and loop fastener.

10. Apparatus in accordance with any of the preceding claims,
**characterized in that** the back element (30, 30') comprises a greater height (HR) than the waist belt (20).

11. Apparatus in accordance with any of the preceding claims,
**characterized in that**, between an upper section (32) of the back element (30) and the first waist belt section (40a) and/or the second waist belt section (40b), a support belt (60) is arranged which is adjustable, in particular, in length, preferably **in that** a free end (61) of it is fed through a loop (33) arranged on the back element (30), wherein the free end (61) can be secured in various positions on an attachment section (63) of the support belt (60), preferably by means of a hook and loop fastener.

12. Apparatus in accordance with any of the preceding claims,
**characterized in that** an upper back element (70) and/or two shoulder belts (80) and/or two leg belts (90) are arranged, in particular, detachably, on the apparatus (10').

## Revendications

1. Dispositif (10, 10') d'assistance pour le transport de charges comprenant une ceinture de hanche (20) ayant un côté intérieur (20a) pour s'appuyer contre le corps d'une personne (100) et un côté extérieur (20b), une première extrémité (21) et une seconde extrémité (22) qui se relient l'une à l'autre avec un élément de fermeture (25),
- la ceinture de hanche (20) ayant un élément de dossier (30, 30') pour s'appuyer contre un premier segment du dos de la personne (100), et
- l'élément de dossier (30, 30') ayant un premier segment de ceinture de hanche (40a),
dispositif **caractérisé en ce que**
le côté extérieur (20a) du premier segment de ceinture de hanche (40a) comporte au moins un et de préférence plusieurs premiers éléments de dépose (50a) à une distance (A) de l'élément de dossier (30, 30') telle que lors de l'appui contre le corps de la personne (100), il est latéralement contre le corps de la personne (100).

2. Dispositif selon la revendication 1,
**caractérisé en ce que**
l'élément de dossier (30, 30') a un second segment de ceinture de hanche (40b),
- le côté extérieur (20b) du second segment de ceinture de hanche (40b) ayant au moins un, et de préférence plusieurs, seconds éléments de dépose (50b) en saillie vers l'extérieur, à une distance (A) de l'élément de dossier (30, 30') telle que lors de l'appui contre le corps de la personne (100), il est latéralement contre le corps de la personne (100) et notamment sur l'autre côté que celui du premier élément de dépôt (50a).

3. Dispositif selon l'une des revendications précédentes,
**caractérisé en ce que**
l'élément de dépose (50a, 50b) est en une mousse dure, notamment en une mousse dure ayant une dureté supérieure à 55 Shore.

4. Dispositif selon l'une des revendications précédentes,
**caractérisé en ce que**
l'élément de dépose (50a, 50b) est installé de manière amovible sur le segment de ceinture de hanche (40a, 40b).

5. Dispositif selon l'une des revendications précédentes,
**caractérisé en ce que**
l'élément de dépose (50a, 50b) est suspendu par un crochet dans un évidement correspondant du segment de ceinture de hanche (40a, 40b), et
- l'élément de dépose (50a, 50b) est fixé à l'aide d'un guide en queue d'aronde à un segment de ceinture de hanche (40a, 40b).

6. Dispositif selon l'une des revendications précédentes,
**caractérisé en ce que**
l'élément de fermeture (25) est une fermeture à griffes.

7. Dispositif selon l'une des revendications précédentes,
**caractérisé en ce que**
la ceinture de hanche (20) est de longueur réglable.

8. Dispositif selon l'une des revendications précédentes,
**caractérisé en ce que**
au moins le premier segment de ceinture de hanche (40a), de préférence le premier segment de ceinture de hanche (40a) et le second segment de ceinture de hanche (40b) sont installés de manière amovible sur l'élément de dossier (30, 30').

9. Dispositif selon l'une des revendications précédentes,
**caractérisé en ce que**
au moins le premier segment de ceinture de hanche (40a), de préférence le premier segment de ceinture de hanche (40a) et le second segment de ceinture de hanche (40b) passent avec une première extrémité (41) à travers un passant (31), de l'élément de dossier (30, 30'),
- l'extrémité libre (41) pouvant se fixer dans des positions différentes à un premier segment de fixation (42) du segment de ceinture de hanche (40a, 40b) de préférence par une liaison à griffes.

10. Dispositif selon l'une des revendications précédentes,
**caractérisé en ce que**
l'élément de dossier (30, 30') a une hauteur (HR) plus grande que celle de la ceinture de hanche (20).

11. Dispositif selon l'une des revendications précédentes,
**caractérisé par**
une ceinture d'appui (60) entre un segment supérieur (32) de l'élément de dossier (30) et le premier segment de ceinture de hanche (40a) et/ou le second segment de ceinture de hanche (40b), cette ceinture d'appui étant réglable notamment dans sa longueur, de préférence en ce qu'elle passe avec son extrémité libre (61) par une boucle (33) prévue sur l'élément de dossier (30),
- l'extrémité libre (61) pouvant se fixer dans des positions différentes au segment de fixation (63) de la ceinture d'appui (60), de préférence avec une liaison à griffes.

12. Dispositif selon l'une des revendications précédentes,
**caractérisé en ce que**
un élément de dossier (70), haut et/ou deux éléments de ceinture d'épaule (80) et/ou de jambière (90) sont installés notamment de manière amovible sur le dispositif (10').
